# EUROPEAN PATENT SPECIFICATION

(11) **EP 3 860 606 B1**
(45) Date of publication and mention of the grant of the patent: **08.11.2023**
(21) Application number: 19779488.6
(22) Date of filing: 01.10.2019
(51) Int. Cl.: A61K 31/47, A61K 47/02, A61K 9/16, A61K 9/48

(54) **PHARMACEUTICAL COMPOSITION COMPRISING LENVATINIB ESYLATE OR TOSYLATE**
PHARMAZEUTISCHE ZUSAMMENSETZUNG MIT LENVATINIB ESYLAT ODER TOSYLAT
COMPOSITION PHARMACEUTIQUE COMPRENANT ESYLATE OU TOSYLATE DE LENVATINIB

(30) Priority: 04.10.2018 EP 18198662
(43) Date of publication of application: 11.08.2021
(73) Proprietor: Synthon B.V., 6545 CM Nijmegen (NL)
(72) Inventor: NOGUEIRAS NIETO, Luis, 08830 Sant Boi de Llobregat (ES); ALVAREZ FERNANDEZ, Lisardo, 08830 Sant Boi de Llobregat (ES)
(74) Representative: Tejedor Vinent, Henar
(86) International application number: PCT/EP2019/076625
(87) International publication number: WO 2020/070147

(56) References cited:
- EP-A1- 1 698 623
- EP-A1- 1 938 842
- EP-A1- 2 468 281
- WO-A1-2017/221214

## Description

### BACKGROUND OF THE PRESENT INVENTION

The present invention relates to a pharmaceutical composition, comprising lenvatinib esylate or tosylate, having improved bioavailability, 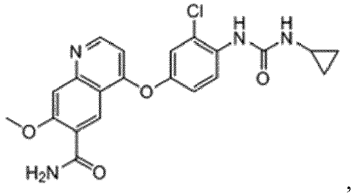 Lenvatinib having the chemical structure shown above is an angiogenesis and C-Kit kinase inhibitor, and as such it is used as a therapeutic agent against various tumors such as thyroid cancer, lung cancer, melanoma and pancreatic cancer.

Lenvatinib and generally pharmaceutically acceptable salts thereof are disclosed in European Patent application EP1415987.

Lenvatinib as such has poor stability under humidifying and warm storage conditions. The marketed product comprises lenvatinib mesylate. It is known that the lenvatinib mesylate salt gelifies when in contact with dissolution media, this may cause a delay in its release. EP1797881 discloses a pharmaceutical composition that solved the above problems using an alkaline excipient with a pH of 8 a 5%w/w aqueous solution to reduce the degradation of the active substance and silicic acid to inhibit gelation. Sodium carbonates are described as suitable alkaline excipient.

EP2468281 discloses that when an alkaline earth metal carbonate is used as a base in combination with a disintegrant the pharmaceutical compositions have superior dissolution properties than other bases, even after long term storage. During prosecution the applicant provided results showing that when non earth metal carbonates such as sodium bicarbonate were used as stabilizers, the dissolution rate decreased after storage as compared to before storage and the dissolution time was additionally delayed.

There is still need of finding additional oral formulations of lenvatinib which overcome the problems of gelation and degradation and is bioequivalent to the commercial lenvatinib mesylate capsules (Lenvima^{®}).

### DETAILED DESCRIPTION OF THE PRESENT INVENTION

The present invention relates to a pharmaceutical composition comprising a therapeutically effective dose of lenvatinib esylate or tosylate and sodium carbonates wherein the weight ratio of lenvatinib esylate or tosylate to sodium carbonates ranges from 1: 1.5 to 1:10, more preferably from 1:2 to 1:7 most preferred ranges are from 1:3 to 1:5.

Sodium carbonates within this invention encompass sodium carbonate (Na₂CO₃), sodium bicarbonate (NaHCO₃), or mixtures of both, for instance effersoda which is sodium bicarbonate coated with sodium carbonate. A preferred carbonate within the invention is sodium bicarbonate.

The inventors have found that surprisingly, formulations comprising lenvatinib esylate or tosylate and sodium carbonates, wherein the weight ratio of lenvatinib esylate or tosylate to sodium carbonates ranges from 1: 1.5 to 1:10, more preferably from 1:2 to 1:7 most preferred ranges are from 1:3 to 1:5, are stable and are bioequivalent to the commercial lenvatinib mesylate capsules (Lenvima^{®}).

Lenvatinib esylate or tosylate present polymorphism and may form solvates, the polymorph or solvates are also included in the present invention. For instance, the tosylate has different polymorphs, form I or form A, all these forms can be used according to the invention.

In the acidic environment of the gastrointestinal tract, sodium carbonates produce CO₂:

NaHCO₃ + HCl → NaCl + H₂O + CO₂

Na₂CO₃ + 2HCl → 2NaCl + H₂O + CO₂

The CO₂ produced has a disintegrant effect that allows lenvatinib esylate or tosylate to disperse in very small fine particles avoiding gelation. The alkaline nature of the sodium carbonates prevents degradation and reduces impurity formation, including genotoxic impurities that may be formed upon hydrolysation of lenvatinib esylate or tosylate. Moreover, the inventors have found that surprisingly, when the sodium carbonates are used in the ratio of the invention, the stability and the disintegration properties of these carbonates are such that lenvatinib esylate or tosylate can be formulated without the addition of an extra disintegrant, as described in EP2468281. The addition of an extra disintegrant is optional and the skilled person could decide to add it.

Furthermore, sodium carbonates and specially NaHCO₃, have better solubility in water than the earth metal carbonates described in EP2468281 improving the pharmaceutical processability i.e. wet granulation with water.

The processability is improved in such a way that no binder is needed in such formulations. The addition of a binder is optional.

In one embodiment, the present invention relates to a pharmaceutical composition comprising a therapeutically effective dose of lenvatinib esylate or tosylate having a particle size distribution D₉₀ from 5 to 50 µm, preferably from 8 to 25 µm, most preferred from 10 to 15 µm.

The D₉₀ value of the particle size distribution is defined as the particle diameter at which 90% by volume of the particles have a smaller diameter than the diameter which corresponds to the D₉₀ value measured by laser diffractometry. Specifically, a Malvern Instruments Mastersizer was used to determine the particle size distribution.

Besides sodium carbonates one or more pharmaceutically acceptable excipients can be used additionally in accordance with the present invention.

In a preferred embodiment sodium carbonates are used in an amount of 14% to 65%, preferably 20% to 55%, more preferably 20% to 50%, most preferably 25% to 50% by weight based on the total weight of the composition.

The one or more pharmaceutically acceptable excipients to be used additionally to sodium carbonates in accordance with the present invention can be chosen from, for example, diluents, binders, disintegrants, lubricants, and glidants.

Diluents are fillers which are used to increase the bulk volume of a tablet or capsule. By combining a diluent with the active pharmaceutical ingredient, the final product is given adequate weight and size to assist in production and handling. Binders hold the excipients that are present in a tablet/granule together.

The pharmaceutical composition of the present invention preferably contains at least one diluent.

Diluents are preferably used in an amount of from 15% to 75%, preferably 30% to 70%, more preferably 35% to 65%, even more preferably 35% to 55% by weight based on the total weight of the composition. Suitable examples of diluents to be used in accordance with the present invention include starch, pregelatinized starch, microcrystalline cellulose (MCC), mannitol, and calcium phosphate.

In a preferred embodiment of the present invention, the diluents to be used are mannitol, microcrystalline cellulose or mixtures thereof.

The pharmaceutical composition of the present invention may also contain a binder. Binders ensure that tablets and granules can be formed having the desired or required mechanical strength. Binders which are suitable for use in accordance with the present invention include povidone, hydroxypropyl cellulose, hydroxypropyl methylcellulose, and sodium carboxyl methylcellulose. Binders are preferably used in an amount of from 1% to 6% by weight based on the total weight of the composition.

The pharmaceutical composition of the present invention may also contain an extra disintegrant. Disintegrants are added to a tablet or capsule composition to promote the breakup of the tablet/capsule into smaller fragments in an aqueous environment, thereby increasing the available surface area and promoting a more rapid release of the active pharmaceutical ingredient. Suitable examples of disintegrants to be used in accordance with the present invention include crospovidone, L-HPC (Low substituted hydroxypropyl cellulose), sodium starch glycolate, croscarmellose sodium, and mixtures of any of the foregoing. Extra disintegrants preferably are used in an amount of from 1% to 25% by weight based on the total weight of the composition; the amount will depend on the tablet size and the chosen disintegrant. A preferred extra disintegrant is low substituted hydroxypropyl cellulose, in a preferred amount of from 15% to 25% by weight based on the total weight of the composition.

The pharmaceutical composition of the invention may also contain a lubricant. Lubricants are generally used in order to reduce sliding friction. In particular, to decrease the friction at the interface between the blend to be encapsulated and dosator of the encapsulation machine. Suitable lubricants to be used in accordance with the present invention include magnesium stearate, stearic acid, glyceryl behenate, hydrogenated vegetable oil, talc and glycerine fumarate. A preferred lubricant is talc. The pharmaceutical composition of the invention may also contain a glidant. Glidants enhance product flow by reducing interparticulate friction. A suitable example is colloidal silicon dioxide.

Lubricants and glidants are used in a total amount of from 0.05% to 5% by weight based on the total weight of the composition, preferably from 1% to 5%.

In a preferred embodiment, the pharmaceutical composition of the present invention, wherein the weight ratio of lenvatinib esylate or tosylate to sodium carbonates ranges from 1: 1.5 to 1:10 more preferably from 1:2 to 1:7 most preferred ranges are from 1:3 to 1:5, contains the following ingredients, based on the total weight of the composition:
a. A therapeutically effective dose of lenvatinib esylate or tosylate in an amount of from 4% to 30% by weight, preferably 4% to 25% by weight;
b. Microcrystalline cellulose in an amount of from 10% to 65% by weight, 20% to 65% by weight, more preferably 25% to 55% by weight, even more preferably 27% to 45% by weight;
c. Sodium carbonates, preferably sodium hydrogen carbonate, from 20% to 55% by weight, preferably from 20% to 50% by weight, more preferably 25% to 50% by weight;
d. Optionally, Low substituted hydroxypropyl cellulose in an amount of from 15% to 25% by weight;
e. Mannitol in an amount of from 7% to 18% by weight, preferably 5% to 10% by weight; and
f. From 1% to 5% by weight of a lubricant and a glidants, preferably talc.

In one embodiment of the present invention, the therapeutically effective dose of lenvatinib is 4 mg, 10 mg, 18mg and 24 mg.

The compositions of the present invention can be prepared by direct mixing or granulating the lenvatinib esylate or tosylate with one or more pharmaceutically acceptable excipients, optionally followed by encapsulation, using equipment and methods well-known to the skilled artisan.

In a preferred embodiment the composition is prepared by granulation process. Granulation can be performed by a wet or dry process. The wet granulation uses water or organic solvents or mixtures thereof as granulation liquid. The dry granulation can be performed by processes known as slugging and/or roller compaction.

The pharmaceutically acceptable excipients to be used in accordance with the present invention, can be used only intragranularly, only extragranularly, or both.

In a preferred embodiment the granules of the present invention are prepared by a wet-granulation process comprising the steps:
a. Mixing lenvatinib esylate or tosylate and sodium carbonates, preferably NaHCO₃, wherein the weight ratio of lenvatinib esylate or tosylate to sodium carbonates ranges from 1: 1.5 to 1:10 most preferred ranges are from 1: 3 to 1:5;
b. Add one or more pharmaceutically acceptable excipients to form a mixture;
c. Wet-granulating the resulting mixture;
d. Further mixing the obtained granulate with one or more further pharmaceutically acceptable excipients to form a further mixture;
e. Optionally encapsulating the granules.

The granules of the present invention typically have a particle size distribution D₅₀ of from 200-350 µm, more preferably from 250 to 300 µm.

The present invention also relates to a pharmaceutical composition comprising granulates as described hereinabove in the form of a capsule or a tablet, preferably a capsule.

The pharmaceutical compositions described herein can be made using conventional methods and equipment well-known in the art.

The pharmaceutical compositions of the present invention show an in vitro dissolution profile wherein at least 80% of lenvatinib esylate or tosylate is released at fifteen minutes when the composition is subjected to a dissolution study in 900 ml HCl 0.1N (pH 1) using a USP apparatus II at 50 rpm at 37°C. Preferably, at least 85% of lenvatinib esylate or tosylate is released from the pharmaceutical composition at fifteen minutes. The pharmaceutical composition in accordance with the present invention is bioequivalent to the commercially available lenvatinib mesylate capsules.

The present invention is illustrated by the following Examples.

### EXAMPLES

### Example 1

**Table 1. Pharmaceutical composition of formulation A.**

| **Formulation A** | | |
|---|---|---|
| **Components** | **mg/capsule** | **%** |
| **Intragranular** | | |
| Lenvatinib tosylate | 14.03 | 14.03% |
| Sodium hydrogen carbonate | 33.00 | 33.00% |
| Mannitol | 8.75 | 8.75% |
| Microcrystalline cellulose | 20.61 | 20.61% |

| **Extragranular** | | |
|---|---|---|
| Microcrystalline cellulose | 20.61 | 20.61% |
| Talc | 3.00 | 3.00% |
| **Total weight** | **100.00** | **100.00%** |

7.02 grams of lenvatinib tosylate, 16.5 grams of sodium hydrogen carbonate, 4.38 grams of mannitol and 10.31 grams of microcrystalline cellulose were weighted and sieved through a 0.8 mm mesh for the deagglomeration of the materials. Afterwards, the mentioned components were mixed for 5 minutes in a vessel at 150 rpm by means of an IKA Eurostard stirrer. Then 9 grams of distilled water was added to the blend in the vessel mixing the components at 150 rpm using the former stirrer. The wetted granules obtained were sieved through 2.8 mm mesh and placed in a oven at 40°C under vacuum conditions for 24 hours. The loss on drying of the granules was measured by means of a halogen moisture analyzer. Once the final granules were dried (1), 10.31 grams of microcrystalline cellulose were weighted and sieved through 0.8 mesh and then mixed with the obtained granules (1) for 10 minutes at 72 rpm resulting in a homogenous blend (2). 1.50 grams of talc were weighted and sieved through 0.5 mm and then mixed with the previous blend (2) for 3 minutes at 72 rpm resulting in a homogenous blend (3). The resulting blend (3) was then encapsulated into hypromellose hard capsules size 4.

## Claims

1. A pharmaceutical composition comprising a therapeutically effective dose of lenvatinib esylate or tosylate and sodium carbonates, wherein the weight ratio of lenvatinib esylate or tosylate to sodium carbonates ranges from 1: 1.5 to 1:10.

2. A pharmaceutical composition according to claim 1 wherein the weight ratio of lenvatinib esylate or tosylate to sodium carbonates ranges from 1: 3 to 1:5.

3. A pharmaceutical composition according to claim 1 or 2 wherein sodium carbonates are present in a range from 20% to 55%, by weight based on the total weight of the composition.

4. A pharmaceutical composition according to any one of claims 1 to 3 wherein the sodium carbonates is sodium bicarbonate.

5. A pharmaceutical composition according to any one of claims 1 to 4, wherein the composition further comprises:
a) At least one diluent in an amount of from 15% to 75% by weigh, preferably 30% to 70% based on the total weight of the composition;
b) Optionally, disintegrant in an amount of from 1% to 25% by weight based on the total weight of the composition; and
c) Lubricant in an amount of from 1 to 5% by weight based on the total weight of the composition.

6. A pharmaceutical composition according to claim 5 wherein the diluent is present in an amount of from 35% to 65% by weight based on the total weight of the composition.

7. A pharmaceutical composition according to claim 5 or 6 wherein the diluent is MCC, mannitol or a mixture of both.

8. A pharmaceutical composition according to any one of the previous claims comprising, based on the total weight of the composition:
a) A therapeutically effective dose of lenvatinib esylate or tosylate in an amount of from 4% to 25% by weight;
b) Microcrystalline cellulose in an amount of from 20% to 65% by weight;
c) Sodium carbonates, preferably sodium hydrogen carbonate, in an amount of from 20% to 55%, preferably 20% to 50% by weight;
d) Optionally, low substituted hydropropyl cellulose in an amount of from 15% to 25% by weight;
e) Mannitol in an amount of from 7% to 18%, preferably 5% to 10% by weight; and
f) Talc in an amount of from 1% to 5% by weight.

9. A pharmaceutical composition according to any one of the previous claims prepared by wet granulation process.

10. A pharmaceutical composition according to any one of the claims 1 to 8 prepared by direct mix.

11. A pharmaceutical composition according to claim 9, which process comprises:
a. Mixing lenvatinib esylate or tosylate and sodium carbonates wherein the weight ratio of lenvatinib esylate or tosylate to sodium carbonates ranges from 1: 1.5 to 1:10;
b. Add one or more pharmaceutically acceptable excipients to form a mixture;
c. Wet-granulating the resulting mixture;
d. Further mixing the obtained granulate with one or more further pharmaceutically acceptable excipients to form a further mixture;
e. Optionally encapsulating the granules.

12. A pharmaceutical composition according to any one of the previous claims in the form of a capsule.

## Patentansprüche

1. Pharmazeutische Zusammensetzung, die eine therapeutisch wirksame Dosis von Lenvatinib-Esylat oder -Tosylat und Natriumcarbonate umfasst, wobei das Gewichtsverhältnis von Lenvatinib-Esylat oder -Tosylat zu Natriumcarbonaten im Bereich von 1:1,5 bis 1:10 liegt.

2. Pharmazeutische Zusammensetzung nach Anspruch 1, wobei das Gewichtsverhältnis von Lenvatinib-Esylat oder -Tosylat zu Natriumcarbonaten im Bereich von 1:3 bis 1:5 liegt.

3. Pharmazeutische Zusammensetzung nach Anspruch 1 oder 2, wobei Natriumcarbonate in einem Bereich von 20 Gew.-% bis 55 Gew.-% basierend auf dem Gesamtgewicht der Zusammensetzung vorhanden sind.

4. Pharmazeutische Zusammensetzung nach einem beliebigen der Ansprüche 1 bis 3, wobei die Natriumcarbonate Natriumbicarbonat sind.

5. Pharmazeutische Zusammensetzung nach einem beliebigen der Ansprüche 1 bis 4, wobei die Zusammensetzung des Weiteren umfasst:
a) mindestens ein Verdünnungsmittel in einer Menge von 15 Gew.-% bis 75 Gew.-%, vorzugsweise 30% bis 70%, basierend auf dem Gesamtgewicht der Zusammensetzung;
b) gegebenenfalls Sprengmittel in einer Menge von 1 Gew.-% bis 25 Gew.-% basierend auf dem Gesamtgewicht der Zusammensetzung; und
c) Gleitmittel in einer Menge von 1 Gew.-% bis 5 Gew.-% basierend auf dem Gesamtgewicht der Zusammensetzung.

6. Pharmazeutische Zusammensetzung nach Anspruch 5, wobei das Verdünnungsmittel in einer Menge von 35 Gew.-% bis 65 Gew.-% basierend auf dem Gesamtgewicht der Zusammensetzung vorhanden ist.

7. Pharmazeutische Zusammensetzung nach Anspruch 5 oder 6, wobei das Verdünnungsmittel MCC, Mannitol oder ein Gemisch aus beiden ist.

8. Pharmazeutische Zusammensetzung nach einem beliebigen der voranstehenden Ansprüche, umfassend, basierend auf dem Gesamtgewicht der Zusammensetzung:
a) eine therapeutisch wirksame Dosis von Lenvatinib-Esylat oder -Tosylat in einer Menge von 4 Gew.-% bis 25 Gew.-%;
b) mikrokristalline Cellulose in einer Menge von 20 Gew.-% bis 65 Gew.-%;
c) Natriumcarbonate, vorzugsweise Natriumhydrogencarbonat, in einer Menge von 20 Gew.-% bis 55 Gew.-%, vorzugsweise 20 Gew.-% bis 50 Gew.-%;
d) gegebenenfalls niedrig substituierte Hydropropylcellulose in einer Menge von 15 Gew.-% bis 25 Gew.-%;
e) Mannitol in einer Menge von 7 Gew.-% bis 18 Gew.-%, vorzugsweise 5 Gew.-% bis 10 Gew.-%; und
f) Talk in einer Menge von 1 Gew.-% bis 5 Gew.-%.

9. Pharmazeutische Zusammensetzung nach einem beliebigen der voranstehenden Ansprüche, hergestellt durch Nassgranulierungsverfahren.

10. Pharmazeutische Zusammensetzung nach einem beliebigen der Ansprüche 1 bis 8, hergestellt durch Direktmischung.

11. Pharmazeutische Zusammensetzung nach Anspruch 9, welches Verfahren umfasst:
a. Mischen von Lenvatinib-Esylat oder -Tosylat und Natriumcarbonaten, wobei das Gewichtsverhältnis von Lenvatinib-Esylat oder -Tosylat zu Natriumcarbonaten im Bereich von 1:1,5 bis 1:10 liegt;
b. Hinzugeben eines oder mehrerer pharmazeutisch verträglicher Hilfsstoffe, um ein Gemisch zu bilden;
c. Nassgranulieren der sich ergebenden Mischung;
d. weiteres Mischen des erhaltenen Granulats mit einem oder mehreren weiteren pharmazeutisch verträglichen Hilfsstoffen, um ein weiteres Gemisch zu bilden;
e. gegebenenfalls Verkapseln der Körner.

12. Pharmazeutische Zusammensetzung nach einem beliebigen der voranstehenden Ansprüche in der Form einer Kapsel.

## Revendications

1. Une composition pharmaceutique comprenant une dose thérapeutiquement efficace d'ésylate ou de tosylate et de carbonates de sodium dans laquelle le rapport pondéral ésylate ou tosylate de lenvatinib/carbonates de sodium est compris entre 1/1,5 et 1/10.

2. Une composition pharmaceutique selon la revendication 1, dans laquelle le rapport pondéral d'ésylate ou de tosylate de lenvatinib/carbonates de sodium est compris entre 1/3 et 1/5.

3. Une composition pharmaceutique selon la revendication 1 ou 2, dans laquelle les carbonates de sodium sont présents en un proportion de 20% à 55% en poids par rapport au poids total de la composition.

4. Une composition pharmaceutique selon l'une quelconque des revendications 1 à 3, dans laquelle les carbonates de sodium consistent en bicarbonate de sodium.

5. Une composition pharmaceutique selon l'une quelconque des revendications 1 à 4, laquelle composition comprend en outre :
a) au moins un diluant en une proportion comprise entre 15% et 75% en poids, de préférence comprise entre 30% et 70% exprimée par rapport au poids total de la composition;
b) éventuellement, un désintégrant en une proportion comprise entre 1% et 25% en poids exprimée par rapport au poids total de la composition; et
c) un lubrifiant en une proportion de 1 à 5% en poids exprimée par rapport au poids total de la composition.

6. Une composition pharmaceutique selon la revendication 5, dans laquelle le diluant est présent en une proportion comprise entre 35% et 65% en poids exprimée par rapport au poids total de la composition.

7. Une composition pharmaceutique selon la revendication 5 ou 6 dans laquelle le diluant est le MCC, le mannitol ou un mélange des deux.

8. Une composition pharmaceutique selon l'une quelconque des revendications précédentes, comprenant, sur la base du poids total de la composition :
a) une dose thérapeutiquement efficace d'ésylate ou de tosylate de lenvatinib en une proportion comprise entre 4% et 25% en poids;
b) une cellulose microcristalline en une proportion comprise entre 20% et 65% en poids;
c) des carbonates de sodium, de préférence l'hydrogénocarbonate de sodium, en une proportion de 20% à 55%, de préférence 20% à 50% en poids ;
d) optionnellement, en une proportion de 15% à 25% en poids d'hydropropylcellulose faiblement substituée;
e) mannitol en une proportion de 7% à 18%, de préférence de 5% à 10% en poids; et
f) talc en une proportion de 1% à 5% en poids.

9. Une composition pharmaceutique selon l'une quelconque des revendications précédentes, préparée par un procédé de granulation par voie humide.

10. Une composition pharmaceutique selon l'une quelconque des revendications 1 à 8, préparée par mélange direct.

11. Une composition pharmaceutique selon la revendication 9, dont le procédé comprend :
a. le mélange de carbonates de sodium et d'ésylate ou de tosylate de lenvatinib dans lequel le rapport pondéral ésylate ou tosylate de lenvatinib/carbonates de sodium est compris entre l/1,5 et 1/10;
b. l'ajout de un ou plusieurs excipients pharmaceutiquement acceptables pour former un mélange;
c. la granulation par voie humide du mélange obtenu;
d. ensuite mélanger le granulat obtenu avec un ou plusieurs autres excipients pharmaceutiquement acceptables pour former un autre mélange;
e. éventuellement l'encapsulation des granulés.

12. Une composition pharmaceutique selon l'une quelconque des revendications précédentes, sous forme de gélules.
